(19) Europäisches Patentamt
European Patent Office
Office européen des brevets

(11) Veröffentlichungsnummer: **0 010 156**
**B1**

(12) # EUROPÄISCHE PATENTSCHRIFT

(45) Veröffentlichungstag der Patentschrift: **06.10.82**

(51) Int. Cl.³: **C 07 D 237/04**, A 61 K 31/50

(21) Anmeldenummer: **79103396.2**

(22) Anmeldetag: **12.09.79**

(54) **6-Arylpyridazin-3-one, diese enthaltende pharmazeutische Zubereitungen und Verfahren zu ihrer Herstellung.**

(30) Priorität: **19.10.78 DE 2845456**

(43) Veröffentlichungstag der Anmeldung: **30.04.80 Patentblatt 80/9**

(45) Bekanntmachung des Hinweises auf die Patenterteilung: **06.10.82 Patentblatt 82/40**

(84) Benannte Vertragsstaaten: **AT BE CH DE FR GB IT NL SE**

(56) Entgegenhaltungen:
**DE-A-1 670 683**
**DE-A-2 207 517**
**DE-A-2 810 267**
**US-A-3 689 652**
**US-A-3 840 662**

**Die Akte enthält technische Angaben, die nach dem Eingang der Anmeldung eingereicht wurden und die nicht in dieser Patentschrift enthalten sind.**

(73) Patentinhaber: **Merck Patent Gesellschaft mit beschränkter Haftung, Frankfurter Strasse 250, D-6100 Darmstadt (DE)**

(72) Erfinder: **Schacht, Erich, Dr., Im Böhl 2, D-6104 Seeheim (DE)**
Erfinder: **Kurmeier, Hans-Adolf, Dr., Heidelberger Strasse 37, D-6100 Darmstadt (DE)**
Erfinder: **Gante, Joachim, Dr., Stormstrasse 4, D-6100 Darmstadt-Arheilgen (DE)**
Erfinder: **Lissner, Reinhard, Dr., Lippmannweg 23, D-6101 Fischbachtal-Lichtenberg (DE)**
Erfinder: **Melzer, Guido, Dr., Mörikestrasse 6, D-6138 Hofheim (DE)**
Erfinder: **Orth, Dieter, Dr., Jahnstrasse 83, D-6100 Darmstadt (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 010 156 B1**

BUNDESDRUCKEREI BERLIN

# 6-Arylpyridazin-3-one, diese enthaltende pharmazeutische Zubereitungen und Verfahren zu ihrer Herstellung

Die Erfindung betrifft neue 6-Arylpyridazin-3-one der allgemeinen Formel I

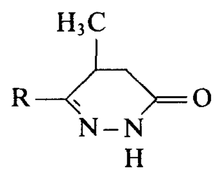

(I)

worin

R einen unsubstituierten oder einen in 4'-Stellung durch F substituierten 4-Biphenylyl-rest bedeutet.

Der Erfindung lag die Aufgabe zugrunde, neue Verbindungen mit wertvollen Eigenschaften aufzufinden, insbesondere solche, die zur Herstellung von Arzneimitteln verwendet werden können. Diese Aufgabe wurde durch die Bereitstellung der Verbindungen der Formel I gelöst.

Es wurde gefunden, daß die Verbindungen der Formel I bei guter Verträglichkeit wertvolle pharmakologische Eigenschaften besitzen. Insbesondere zeigen sie antiarteriosklerotische und lipidspiegelsenkende Wirkungen. So wirken sie cholesterinspiegelsenkend (nachweisbar im Serum von Ratten nach der Methode von Levine et al., Automation in Analytical Chemistry, Technicon Symposium 1967, Mediad, New York, Seiten 25—28) und triglyceridspiegelsenkend (nachweisbar nach der Methode von Noble und Campbell, Clin. Chem. 16 [1970], Seiten 166—170). Weiterhin treten antithrombotische, vor allem thrombozytenaggregationshemmende Eigenschaften auf.

Die Beeinflussung der Thrombozytenfunktion im Sinne einer Aggregationshemmung kann an Kaninchen im Born-Test in vitro und ex vivo (Nature, 194 [1962], Seiten 927—929) und im Faser-Test nach Jacobi (Thrombos. Diathes. haemorrh. 26 [1971], Seiten 192—202) nachgewiesen werden. Außerdem treten antiphlogistische, fibrinolytische, blutzuckersenkende und analgetische Eigenschaften sowie Wirkungen auf das Zentralnervensystem auf, die nach hierfür geläufigen Methoden ermittelt werden können.

Die Verbindungen der Formel I können daher als Arzneimittel in der Human- und Veterinärmedizin verwendet werden. Ferner können sie als Zwischenprodukte zur Herstellung weiterer Arzneimittelwirkstoffe verwendet werden.

Gegenstand der Erfindung sind die 6-Arylpyridazin-3-one der Formel I.

Gegenstand der Erfindung ist ferner ein Verfahren zur Herstellung der 6-Arylpyridazin-3-one der Formel 1, dadurch gekennzeichnet, daß man eine Carbonsäure der allgemeinen Formel II

$$R-CO-CH-(CH_3)-CH_2-COOH \qquad II$$

worin

R die bei Formel I angegebene Bedeutung hat,

oder ein reaktionsfähiges Derivat einer solchen Carbonsäure mit Hydrazin umsetzt.

Die Herstellung der Verbindungen der Formel I erfolgt im übrigen nach an sich bekannten Methoden, wie sie in der Literatur (z. B. in den Standardwerken wie Houben-Weyl, Methoden der Organischen Chemie, Georg-Thieme-Verlag, Stuttgart) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Die Ketosäuren der Formel II können nach an sich bekannten Methoden hergestellt werden, beispielsweise durch Umsetzung von Biphenylen der Formel R-H mit Methyl-bernsteinsäureanhydrid nach der Methode von Friedel-Crafts in Gegenwart von $AlCl_3$.

Anstelle der Carbonsäuren der Formel II können auch ihre reaktionsfähigen Derivate in die erfindungsgemäße Reaktion eingesetzt werden. Als solche eignen sich insbesondere die Ester, z. B. die Alkylester, worin die Alkylgruppe vorzugsweise 1—4 C-Atome besitzt, insbesondere die Methyl- und Äthylester. Weiterhin können z. B. die Säurehalogenide der Säuren der Formel II verwendet werden, z. B. die Säurechloride oder Säurebromide. Weitere geeignete reaktionsfähige Derivate der Carbonsäuren der Formel II können während der Reaktion in situ gebildet werden, ohne daß man sie isoliert. Dazu gehören beispielsweise die Hydrazone der Formel

$$R-C(=N-NH_2)-CH(CH_3)-CH_2-COOH$$

die Hydrazide der Formel

$$R-CO-CH(CH_3)-CH_2-CO-NH-NH_2$$

und die Hydrazone dieser Hydrazide der Formel

$$R—C(=N—NH_2)—CH(CH_3)—CH_2—CO—NH—NH_2$$

Auch die übrigen Ausgangsstoffe können gewünschtenfalls in situ gebildet werden, derart, daß man sie aus dem Reaktionsgemisch nicht isoliert, sondern sofort weiter zu den Verbindungen der Formel I umsetzt.

Für die Umsetzung mit den Carbonsäuren der Formel II verwendet man vorteilhaft einen Überschuß Hydrazin, das gleichzeitig als Lösungsmittel dienen kann. Zweckmäßiger ist es jedoch, ein zusätzliches inertes Lösungsmittel zuzusetzen. Als inerte Lösungsmittel eignen sich vorzugsweise Alkohole wie Methanol, Äthanol, Isopropanol, n-Butanol, Isoamylalkohol, Glykole und deren Äther wie Äthylenglykol, Diäthylenglykol, Äthylenglykolmonomethyl- oder -monoäthyläther (Methylglykol), ferner Äther, insbesondere wasserlösliche Äther wie Tetrahydrofuran, Dioxan oder Äthylenglykoldimethyläther (Diglyme); ferner Wasser sowie Gemische dieser Lösungsmittel untereinander, insbesondere Gemische mit Wasser, z. B. wässeriges Äthanol. Die Reaktionstemperaturen liegen zweckmäßig zwischen etwa 20 und etwa 200°, vorzugsweise zwischen 60 und 80°, die Reaktionszeiten zwischen etwa 1 und 3 Stunden.

Die Verbindungen der Formel I besitzen ein Asymmetriezentrum. Sie können daher bei ihrer Herstellung als Racemate oder, falls optisch aktive Ausgangsstoffe verwendet werden, auch in optisch aktiver Form erhalten werden. Erhaltene Racemate können, falls erwünscht, nach an sich bekannten Methoden mechanisch oder chemisch in ihre optischen Antipoden getrennt werden.

Gegenstand der Erfindung ist ferner die Verwendung der Verbindungen der Formel I zur Herstellung pharmazeutischen Zubereitungen, insbesondere auf nicht-chemischem Wege. Hierbei können sie zusammen mit mindestens einem festen, flüssigen oder halbflüssigen Träger- oder Hilfsstoff und gegebenenfalls zusammen mit einem oder mehreren weiteren Wirkstoff(en) in eine geeignete Dosierungsform gebracht werden.

Gegenstand der Erfindung sind ferner Mittel, insbeondere pharmazeutische Zubereitungen, enthaltend eine Verbindung der Formel I.

Diese Zubereitungen können als Arzneimittel in der Human- oder Veterinärmedizin verwendet werden. Als Trägerstoffe kommen organische oder anorganische Substanzen in Frage, die sich für die enterale (z. B. orale), parenterale oder topikale Applikation eignen und mit den neuen Verbindungen nicht reagieren, beispielsweise Wasser, pflanzliche Öle, Benzylalkohole, Polyäthylenglykole, Glycerintriacetat, Gelatine, Kohlehydrate wie Lactose oder Stärke, Magnesiumstearat, Talk, Vaseline. Zur oralen Anwendung dienen insbesondere Tabletten, Dragees, Kapseln, Sirupe, Säfte oder Tropfen, zur rektalen Anwendung Suppositorien, zur parenteralen Anwendung Lösungen, vorzugsweise ölige oder wäßrige Lösungen, ferner Suspensionen, Emulsionen oder Implantate, für die topikale Anwendung Salben, Cremes oder Puder. Sofern die Arzneimittel in Form abgeteilter Pulver verabreicht werden sollen, so sind auch die Verpackungsmaterialien wie Papierbriefchen oder Papierkapseln geeignete Trägerstoffe. Die neuen Verbindungen können auch lyophilisiert und die erhaltenen Lyophilisate z. B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel,Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z. B. ein oder mehrere Vitamine.

Insbesondere eignen sich die Verbindungen der Formel I zur Behandlung und/oder Prophylaxe von Krankheitsbildern mit erhöhten Werten der Serumlipide und Thromboseneigung, von primären und sekundären Hyperlipoproteinämien mit und ohne Xanthomatose, von Atherosklerose (Koronarsklerose, Zerebralsklerose, periphere Gefäßsklerose), von diabetischen Angiopathien (diabetische Retinopathie).

Die erfindungsgemäßen Substanzen werden in der Regel in Analogie zu bekannten, im Handel befindlichen Lipidsenkern (z. B. Clofibrat) verabreicht, vorzugsweise in Dosierungen zwischen etwa 10 und 1000 mg, insbesondere zwischen 50 und 500 mg pro Dosierungseinheit. Die tägliche Dosierung liegt vorzugsweise zwischen etwa 0,2 und 100 mg/kg Körpergewicht. Die besondere Dosis für jeden bestimmten Patienten hängt jedoch von den verschiedensten Faktoren ab, beispielsweise von der Wirksamkeit der eingesetzten speziellen Verbindung, vom Alter, Körpergewicht, dem allgemeinen Gesundheitszustand, dem Geschlecht, von der Kost, vom Verabfolgungszeitpunkt und -weg, von der Ausscheidungsgeschwindigkeit, von Arzneistoffkombinationen und der Schwere der jeweiligen Erkrankung, welche die Therapie gilt. Die orale Applikation ist bevorzugt.

Vor- und nachstehend sind die Temperaturen in Celsiusgraden angegeben.

## Beispiel 1

Man löst 28,6 g 4-(4′-Fluor-4-biphenylyl)-4-oxo-3-methyl-buttersäure in 430 ml Äthanol, gibt 9 ml 80%iges Hydrazinhydrat hinzu und kocht 3 Stunden. Anschließend kühlt man ab, saugt das

ausgefallene 5-Methyl-6-(4'-fluor-4-biphenylyl)-2,3,4,5-tetrahydropyridazin-3-on ab und wäscht mit wenig Aceton, F. 198—199°.

Beispiel 2

Analog Beispiel 1 erhält man durch Umsetzung der entsprechenden 4-Oxocarbonsäure mit Hydrazin 5-Methyl-6-(4-biphenylyl)-2,3,4,5-tetrahydropyridazin-3-on, F. 212°.

**Patentansprüche für die Vertragsstaaten BE, CH, DE, FR, GB, IT NL, SE**

1. 6-Arylpyridazin-3-one der allgemeinen Formel I

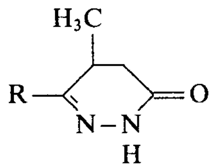

(I)

worin
R einen unsubstituierten oder einen in 4'-Stellung durch F substituierten 4-Biphenylylrest bedeutet.

2. Verfahren zur Herstellung von 6-Arylpyridazinonen der allgemeinen Formel I nach Patentanspruch 1

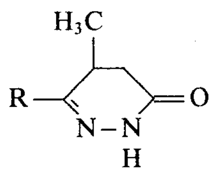

(I)

worin
R einen unsubstituierten oder einen in 4'-Stellung durch F substituierten 4-Biphenylylrest bedeutet, dadurch gekennzeichnet, daß man eine Carbonsäure der allgemeinen Formel II

$$R-CO-CH(CH_3)-CH_2-COOH \qquad II$$

worin
R die bei Formel I angegebene Bedeutung hat, oder ein reaktionsfähiges Derivat einer solchen Carbonsäure mit Hydrazin
umsetzt.

3. Verfahren zur Herstellung pharmazeutischer Zubereitungen, dadurch gekennzeichnet, daß man eine Verbindung der allgemeinen Formel I nach Patentanspruch 1 zusammen mit mindestens einem festen, flüssigen oder halbflüssigen Träger- oder Hilfsstoff und gegebenenfalls zusammen mit einem weiteren Wirkstoff in eine geeignete Dosierungsform bringt.

4. Pharmazeutische Zubereitung, gekennzeichnet durch einen Gehalt an einer Verbindung der allgemeinen Formel I nach Patentanspruch 1.

**Patentanspruch für den Vertragsstaat: AT**

Verfahren zur Herstellung von 6-Arylpyridazinonen der allgemeinen Formel I

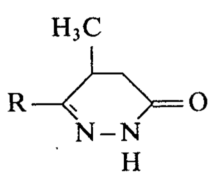

(I)

worin
R einen unsubstituierten oder einen in 4'-Stellung durch F substituierten 4-Biphenylylrest bedeutet,

dadurch gekennzeichnet, daß man eine Carbonsäure der allgemeinen Formel II

$$R-CO-CH(CH_3)-CH_2-COOH \quad II$$

worin

R die bei Formel I angegebene Bedeutung hat, oder ein reaktionsfähiges Derivat einer solchen Carbonsäure mit Hydrazin

umsetzt.

**Claims for the Contracting States: BE, CH, DE, FR, GB, IT, NL, SE**

1. 6-Arylpyridazin-3-ones of the general formula I

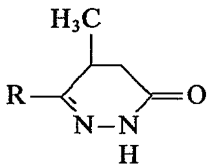

(I)

wherein

R is a 4-biphenylyl radical which is unsubstituted or is substituted in the 4′-position by F.

2. Process for the preparation of 6-arylpyridazinones of the general formula I according to Claim 1

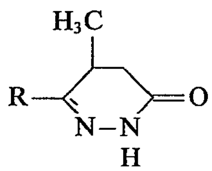

(I)

wherein

R is a 4-biphenylyl radical which is unsubstituted or is substituted in the 4′-position by F, characterised in that a carboxylic acid of the general formula II

$$R-CO-CH(CH_3)-CH_2-COOH \quad II$$

wherein

R has the meaning given for formula I, or a reactive derivative of such a carboxylic acid, is reacted with hydrazine.

3. Process for the preparation of pharmaceutical formulations, characterised in that a compound of the general formula I according to Claim 1, together with at least one solid, liquid or semi-liquid excipient or auxiliary, and, if desired, together with an additional active ingredient, is converted to a suitable dosage form.

4. Pharmaceutical formulations, characterised in that it contains a compound of the general formula I according to Claim 1.

**Claim for the Contracting State: AT**

Process for the preparation of 6-arylpyridazinones of the general formula I according to Claim 1

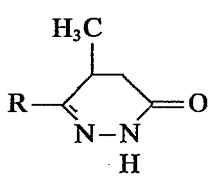

(I)

wherein

R is a 4-biphenylyl radical which is unsubstituted or is substituted in the 4′-position by F, characterised in that a carboxylic acid of the general formula II

$$R-CO-CH(CH_3)-CH_2-COOH \quad II$$

wherein

R has the meaning given for formula I, or a reactive derivative of such a carboxylic acid, is reacted with hydrazine.

**Revendications pour les Etats contractants: BE, CH, DE, FR, GB, IT, NL, SE**

1. 6-Arylpyridazine-3-ones de formule générale I

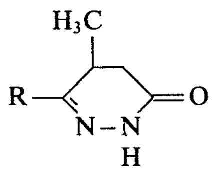

(I)

dans laquelle

R représente un reste 4-biphénylyle non substitué ou substitué en position 4' par F.

2. Procédé de préparation des 6-arylpyridazinones de formule générale I selon la revendication 1

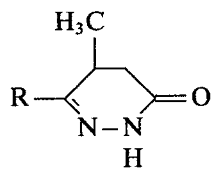

(I)

dans laquelle

R représente un reste 4-biphénylyle non substitué ou substitué en position 4' par F, caractérisé en ce que l'on fait réagir un acide carboxylique de formule générale II

$$R-CO-CH(CH_3)-CH_2-COOH \quad \text{II}$$

dans laquelle

R a la signification indiquée en référence à la formule I, ou un dérivé réactif d'un tel acide carboxylique,

avec l'hydrazine.

3. Procédé de préparation de compositions pharmaceutiques, caractérisé en ce que l'on met sous une forme de dosage appropriée un composé de formule générale I selon la revendication 1 avec au moins un véhicule ou produit auxiliaire solide, liquide ou semiliquide et le cas échéant avec une autre substance active.

4. Composition pharmaceutique caractérisée en ce qu'elle contient un composé de formule générale I selon la revendication 1.

**Revendication pour l'Etat contractant: AT**

Procédé de préparation de 6-arylpyridazinones de formule générale I

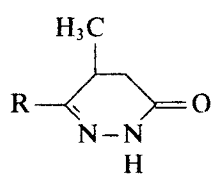

(I)

dans laquelle

R représente un reste 4-biphénylyle non substitué ou substitué en position 4' par F, caractérisé en ce que l'on fait réagier un acide carboxylique de formule générale II

$$R-CO-CH(CH_3)-CH_2-COOH \quad \text{II}$$

dans laquelle

R a la signification indiquée en référence à la formule I, ou un dérivé réactif d'un tel acide carboxylique,

avec l'hydrazine.